Europäisches Patentamt

European Patent Office

Office européen des brevets

(11) Publication number: **0 089 126**
**B1**

(12) **EUROPEAN PATENT SPECIFICATION**

(45) Date of publication of patent specification: 30.07.86

(51) Int. Cl.⁴: **C 12 P 7/02, C 12 P 7/24, C 12 P 7/22**

(21) Application number: 83300884.0

(22) Date of filing: 21.02.83

(54) **Process of oxidising hydrocarbons.**

(30) Priority: 15.03.82 GB 8207497

(43) Date of publication of application:
21.09.83 Bulletin 83/38

(45) Publication of the grant of the patent:
30.07.86 Bulletin 86/31

(84) Designated Contracting States:
BE DE FR GB IT NL SE

(56) References cited:
FR-A-2 215 465
GB-A-2 081 306
US-A-3 880 739

RUSSIAN CHEMICAL REVIEWS, vol. 49, no. 5,
May 1980, pages 385-403, London, G.B. K.
MARTINEK et al.: "The stabilisation of enzymes
- a key factor in the practical application of
biocatalysis"

(73) Proprietor: IMPERIAL CHEMICAL INDUSTRIES
PLC
Imperial Chemical House Millbank
London SW1P 3JF (GB)

(72) Inventor: Taylor, John Anthony
39 Dinsdale Drive Eaglescliffe
Stockton-on-Tees Cleveland (GB)
Inventor: Higgins, Raymond
29 Springfield Stokesley
Middlesbrough Cleveland (GB)

(74) Representative: Locke, Timothy John et al
Imperial Chemical Industries PLC Legal
Department: Patents Bessemer Road PO Box 6
Welwyn Garden City Hertfordshire, AL7 1HD
(GB)

Courier Press, Leamington Spa, England.

## Description

This invention relates to a process of producing alcohols and phenols by oxidising hydrocarbons.

It is known that various enzymes are capable of oxidising hydrocarbons. Enzymes are known which oxidise alkanes to alcohols or which oxidise the alkyl chains of alkyl benzenes to produce aromatic alcohols or which oxidise benzene or alkyl benzenes to produce phenols or alkyl phenols, (hydrocarbon oxygenases). Enzymes are also known which further oxidise alcohols to produce aldehydes (alcohol dehydrogenases).

Suitable enzymes are contained in various species of microorganism and may be used either as the microorganisms themselves or after separation of the enzymes and optionally supporting the enzymes on suitable supports. In order to avoid complications from contamination with other bacterial strains it is desirable to operate in the absence of nutrient media; in consequence any microorganisms present will not increase substantially in numbers.

In order to oxidise hydrocarbons using such enzymes it is necessary to bring them into contact with the enzyme and water in the presence of oxygen. Since hydrocarbons and water are immiscible an organic and an aqueous phase is formed. The oxidation products accumulate in the organic phase. The organic phase may therefore be separated and products recovered from it by any suitable means for example distillation.

We have found it to be advantageous in producing alcohols and phenols using microorganisms containing hydrocarbon hydroxylating oxygenases to provide that an organic phase comprising 20 to 80% of the total volume of the reaction mixture be present, as with this amount of organic phase separation of the organic phase from the aqueous phase containing the enzyme system is facilitated. If the amount of the organic phase is outside the above range an unacceptable proportion of organic material tends to be dispersed in the aqueous phase, for example by absorption on to any microorganism present, or the aqueous phase and any microorganisms contained in it tend to be dispersed into the organic phase. Although the phases may in these events be separated in at least some cases the process can be difficult requiring for example centrifuging or the use of additives.

The invention comprises a process which comprises producing an alcohol or phenol by oxidising a hydrocarbon which is an alkane having 6 to 18 and preferably 10 to 15 carbon atoms which is preferably linear at least as regards a group of 4 terminal carbon atoms, or benzene or an alkyl benzene having at most 10 carbon atoms in its alkyl groups or groups by contacting an organic phase comprising the hydrocarbon with a hydrocarbon hydroxylating oxygenase and water in the presence of oxygen, characterised in that the hydrocarbon hydroxylating oxygenase is contained in a microorganism and in that organic phase constitutes 20 to 80% preferably 30 to 70% and more preferably 40 to 60% by volume of the total reaction mixture, and at least 15%, preferably 15 to 75% and more preferably 20 to 60% of water is present by volume of the total reaction mixture.

In a batch process, operation in accordance with the invention permits the production of a greater amount of product per unit of enzyme present than is produced with smaller amounts of the organic phase.

If desired the organic phase may comprise a substantially inert solvent for example a cyclo alkane or highly branched alkane in an amount for example of 0 to 50%. Preferably however it consists substantially only of the hydrocarbon to be oxidised and oxidation products thereof. Suitably a concentration of at least 0.05% by weight of the product is present in the organic phase, as this aids separation of the product from the phase.

The alkane hydroxylating oxygenase is very suitably provided as a suspension of bacterial cells in water. Suitable bacterial cells are those of Arthrobacter paraffinaeus, ATCC No 21298 Acinetobacter strain NCIB 11613 or Methylococcus capsulatus (Bath strain) NCIB 11132.

Oxygen may be supplied to the reaction as pure oxygen but is very suitably supplied as air.

The pressure at which the reaction is carried out is not critical though pressures of 0.1 to 10 atmospheres, for example atmospheric pressures, are preferred. The partial pressure of oxygen present should be controlled such that inflammable mixtures are not formed.

The process may be carried out at any temperatures under which the oxygenase is stable but temperatures above 20°C are preferred in order to increase reaction rates and facilitate temperature control by cooling. It is particularly preferred to carry out the process at a temperature in the range 25 to 80°C.

The product hydroxylated compounds may be recovered from the organic phase by decanting the organic phase and recovering the alcohols from it by distillation. If unconverted alkane is present it may be recycled to the process.

The process is preferably operated to produce terminal alcohols as these are useful surfactant intermediates. It is preferred therefore that the hydrocarbon-hydroxylating oxygenase should be a terminal alkane hydroxylating mono-oxygenase in order to produce such alcohols. It may also be used for producing alkanals, hydroxybenzenes, for example phenol, or phenyl alkanols.

The process may be carried out in a batch or continuous mode. It is preferred when operating in a continuous mode that the feed rate of hydrocarbon in litres per hour divided by the volume of water present in the reaction mixture in litres should be in the range $10^{-2}$ to 40 $hr^{-1}$. The feed rate is suitably adjusted in accordance with the rate of reaction.

Example 1

A medium was prepared containing mineral salts (composition in Table 1), 0.5% w/v monosodium glutamate and 1% v/v n-dodecane, adjusted to pH 7.0. Inocula of Arthrobacter paraffineous ATCC 21298 cells were added to 200 ml aliquots of this medium and shaken at 30°C for 18 hours. The cells were then harvested by centrifugation and stored as a frozen paste.

The cell paste was thawed, and the cells resuspended in an aqueous phosphate solution, containing 1.56 gm/l $NaH_2PO_4 \cdot 2H_2O$ and 1.90 gm/l of $K_2HPO_4$, adjusted to pH 7.0, to give a cell dry weight of 1.2 gm/l.

50 ml aliquots of cell suspension were shaken at 30°C in separats flasks with various volumes of dodecane. At intervals, samples of the dodecane layer were separated from the cell suspension and dodecan-1-ol was separated from the dodecane by gas liquid chromatography.

The amounts of dodecanol formed were as follows (expressed as milligrams of dodecanol per flask):—

| Time (hours) | Volume of dodecane added | | | |
|---|---|---|---|---|
|  | 50 ml | 25 ml | 10 ml | 5 ml |
| 17 | 0.30 | 0.19 | <0.1 | <0.1 |
| 41 | 1.2 | 0.53 | 0.17 | <0.2 |
| 65 | 1.6 | 0.53 | 0.23 | not determined |
| 89 | 1.6 | 0.63 | 0.23 | 0.15 |
| 161 | 1.5 | 0.48 | 0.19 | 0.12 |

Yields of dodecanol are increased as the quantity of dodecane approaches the volume of the cell suspension.

At the 5 ml level, separation of the dodecane/dodecanol mixture by simple decantation was impractical and the dodecanol was recovered by adding 45 ml of dodecane before decantation. The results given represent four experiments terminating at the specified times but otherwise identical. At the 10, 25 and 50 ml levels of dodecane added in the experiment simple decantation was possible.

Example 2

A medium was prepared containing mineral salts (composition in Table 1) and 1% v/v n-dodecane. Inocula of cells of Acinetobacter S2 (NCIB 11613) were added to 200 ml aliquots of this medium and shaken at 30°C for 18 hours. The cells were then harvested by centrifugation.

The cells were resuspended in the aqueous phosphate buffer solution described in Example 1, to give a (dry) weight of 0.5 gm/l.

25 ml aliquots of this cell suspension were shaken in separate flasks at 30°C with various volumes of n-dodecane. At intervals, samples of the dodecane layer were separated from the cell suspension and dodecan-1-ol was separated from the dodecane by gas liquid chromatography.

The amounts of dodecanol formed were as follows (expressed as milligrams of dodecanol per flask):—

| Time hr | Volume of dodecane ml | | |
|---|---|---|---|
|  | 25 ml | 10 ml | 5 ml |
| 2 | 7.1 | 5.9 | 0.5 |
| 4 | 12.1 | 8.8 | 2.0 |
| 21 | 17.8 | 6.0 | 2.8 |
| 26 | 18.9 | 5.2 | 1.0 |

Simple decantation of the crude product was possible in each case, though a long settling time was required with the 5 ml sample.

Table 1

The mineral salts medium used in Examples 1 and 2 contained the following (concentrations are in gm/l):—

| | |
|---|---|
| $(NH_4)_2\ SO_4$ | : 1.8 |
| $Mg\ SO_4 \cdot 7H_2O$ | : 0.2 |
| $NaH_2PO_4 \cdot 2H_2O$ | : 1.56 |
| $K_2HPO_4$ | : 1.9 |

To this medium is added (in proportions of 1 ml to 1 l of medium) a trace elements solution made up as follows (concentrations in mg/l):—

| | |
|---|---|
| $FeSO_4 \cdot 7H_2O$ | : 500 |
| $CuSO_4 \cdot 5H_2O$ | : 100 |
| $MnSO_4 \cdot 5H_2O$ | : 50 |
| $ZnSO_4 \cdot 7H_2O$ | : 50 |
| $CaCl_2 \cdot 2H_2O$ | : 1320 |
| $CoCl_2$ | : 10 |
| $H_3BO_3$ | : 7 |
| $Na_2MoO_4$ | : 10 |

Example 3

A nitrate minimal medium was prepared as described by R Whittenbury, K C Philips and J F Wilkinson (Journal of General Microbiology, Volume 61, Page 205, 1970). Methyloccus capsulatus, (Bath), NCIB 11132, was grown in a continuous culture apparatus, using this medium. A gaseous mixture comprising methane (50% by volume) and air (50% by volume) was passed through. The medium was fed into the fermenter and culture removed at a rate of 0.02 volumes per volume of fermentation liquid per hour. The cells were harvested by centrifugation and stored as a frozen paste.

The cells paste was thawed, and the cells were resuspended in the aqueous phosphate buffer solution described in Example 1, to give a dry cell weight of 0.5 gm/l.

50 ml aliquots of this cell suspension were shaken in separate flasks at 30°C. At intervals samples of the dodecane layer were separated from the cell suspension, and dodecan-1-ol was separated from the dodecane by gas liquid chromatography.

The amounts of dodecanol formed were as follows (expressed as milligrams of dodecanol per flask):—

| Time hours | Volume of dodecane ml | | | |
|---|---|---|---|---|
| | 50 ml | 25 ml | 10 ml | 5 ml |
| 16.5 | 0.50 | 0.64 | 0.45 | 0.20 |
| 40.5 | 0.88 | 0.43 | 0.16 | 0.08 |
| 64.5 | 0.84 | 0.31 | 0.01 | 0.03 |

Yields of dodecanol are increased as the quantity of dodecane approaches the volume of the cell suspension. At the 5 ml level, separation of the dodecane/dodecanol mixture by simple decantation was impractical and the dodecanol was recovered by adding 45 ml of dodecane before decantation. The results at the 5 ml level represent three experiments terminating at the specified times but otherwise identical. At the 10, 25 and 50 ml levels of dodecane added in the experiment simple decantation was possible.

**Claims**

1. A process which comprises producing an alcohol or phenol by oxidising a hydrocarbon which is an alkane having 6 to 18 carbon atoms or benzene or an alkyl benzene having at most 10 carbon atoms in its alkyl group or groups which comprises contacting an organic phase comprising the hydrocarbon with a hydrocarbon hydroxylating oxygenase and water in the presence of oxygen, characterised in that the hydrocarbon hydroxylating oxygenase is contained in a microorganism and the organic phase constitutes 20 to 80% by volume of the total reaction mixture, at least 15% of water being present by volume of the total reaction mixture.

2. A process according to claim 1 in which the hydrocarbon is an alkane having 10 to 15 carbon atoms and which is linear at least as regards a group of 4 terminal carbon atoms.

3. A process as claimed in claim 1 or 2 in which the organic phase constitutes 30 to 70% by volume of the total reaction mixture and 15 to 75% of water is present by volume of the total reaction mixture.

4. A process as claimed in any preceding claim in which the organic phase consists substantially only of the hydrocarbon to be oxidised and reaction products thereof.

5. A process as claimed in claim 1, 2, 3 or 4 which is carried out as a batch process.

6. A process as claimed in any preceding claim in which the reaction is carried out at a pressure of 0.1 to 10 atmospheres.

7. A process as claimed in any preceding claim which is carried out at a temperature in the range 25 to 80°C.

8. A process as claimed in any preceding claim in which alcohol is recovered by decanting the organic phase and distilling it.

9. A process as claimed in any preceding claim in which the hydrocarbon hydroxylating oxygenase is a terminal alkane hydroxylating mono-oxygenase.

**Patentansprüche**

1. Verfahren, bei dem durch Oxidation eines Kohlenwasserstoffs, der ein Alkan mit 6 bis 18 Kohlenstoffatomen oder Benzol oder ein Alkylbenzol mit höchstens 10 Kohlenstoffatomen in seiner Alkylgruppe oder seinen Alkylgruppen ist, ein Alkohol oder Phenol hergestellt wird, wobei eine organische Phase, die den Kohlenwasserstoff enthält, in Gegenwart von Sauerstoff mit einer die Hydroxylierung von Kohlenwasserstoffen bewirkenden Oxygenase und Wasser in Berührung gebracht wird, dadurch gekennzeichnet, daß die die Hydroxylierung von Kohlenwasserstoffen bewirkende Oxygenase in einem Mikroorganismus enthalten ist und die organische Phase 20 bis 80 Vol.-% der gesamten Reaktionsmischung ausmacht, wobei mindestens 15% Wasser, bezogen auf das Volumen der gesamten Reaktionsmischung, vorhanden sind.

2. Verfahren nach Anspruch 1, bei dem der Kohlenwasserstoff ein Alkan mit 10 bis 15 Kohlenstoffatomen ist, das mindestens in bezug auf eine Gruppe von 4 endständigen Kohlenstoffatomen linear ist.

3. Verfahren nach Anspruch 1 oder 2, bei dem die organische Phase 30 bis 70 Vol.-% der gesamten Reaktionsmischung ausmacht und 15 bis 75% Wasser, bezogen auf das Volumen der gesamten Reaktionsmischung, vorhanden sind.

4. Verfahren nach einem der vorhergehenden Ansprüche, bei dem die organische Phase im wesentlichen nur aus dem zu oxidierenden Kohlenwasserstoff und aus seinen Reaktionsprodukten besteht.

5. Verfahren nach Anspruch 1, 2, 3 oder 4, das als diskontinuierliches Verfahren durchgeführt wird.

6. Verfahren nach einem der vorhergehenden Ansprüche, bei dem die Reaktion unter einem Druck von 0,1 bis 10 Atmosphären durchgeführt wird.

7. Verfahren nach einem der vorhergehenden Ansprüche, das bei einer Temperatur von 25 bis 80°C durchgeführt wird.

8. Verfahren nach einem der vorhergehenden Ansprüche, bei dem Alkohol gewonnen wird, indem die organische Phase dekantiert und destilliert wird.

**0 089 126**

9. Verfahren nach einem der vorhergehenden Ansprüche, bei dem die die Hydroxylierung von Kohlenwasserstoffen bewirkende Oxygenase eine Monooxygenase ist, die die Hydroxylierung von Alkanen unter Bildung endständiger Hydroxylgruppen bewirkt.

**Revendications**

1. Procédé de production d'un alcool ou d'un phénol par oxydation d'un hydrocarbure qui est un alcane ayant 6 à 18 atomes de carbone ou du benzène ou d'un alcoylbenzène ayant au plus 10 atomes de carbone dans son ou ses groupes alcoyles, qui comprend la mise en contact d'une phase organique comprenant l'hydrocarbure avec une oxygénase hydroxylant des hydrocarbures et de l'eau en présence d'oxygène, caractérisé en ce que l'oxygénase hydroxylant les hydrocarbures est contenue dans un micro-organisme et que la phase organique constitue 20 à 80% en volume du mélange réactionnel total qu'il y a au moins 15% d'eau en volume par rapport au mélange réactionnel total.

2. Procédé suivant la revendication 1, caractérisé en ce que l'hydrocarbure est un alcane ayant 10 à 15 atomes de carbone, qui est linéaire au moins en ce qui concerne un groupe de 4 atomes de carbone terminaux.

3. Procédé suivant la revendication 1 ou la revendication 2, caractérisé en ce que la phase organique constitue 30 à 70% en volume du mélange réactionnel total et qu'il y a 15 à 75% d'eau en volume par rapport au mélange réactionnel total.

4. Procédé suivant l'une quelconque des revendications 1 à 3, caractérisé en ce que la phase organique ne comprend essentiellement que l'hydrocarbure à oxyder et ses produits de réaction.

5. Procédé suivant l'une quelconque des revendications 1 à 4, caractérisé en ce qu'il est réalisé en discontinu.

6. Procédé suivant l'une quelconque des revendications 1 à 5, caractérisé en ce que la réaction est effectuée à une pression de 0,1 à 10 atmosphères.

7. Procédé suivant l'une quelconque des revendications 1 à 6, caractérisé en ce qu'il est conduit à une température dans la gamme de 25 à 80°C.

8. Procédé suivant l'une quelconque des revendications 1 à 7, caractérisé en ce que l'alcool est récupéré par décantation de la phase organique et distillation de celle-ci.

9. Procédé suivant l'une quelconque des revendications 1 à 8, caractérisé en ce que l'oxygénase hydroxylant des hydrocarbures est une mono-oxygénase hydroxylant un alcane en position terminale.